**Europäisches Patentamt**

**European Patent Office**

**Office européen des brevets**

(19)

(11) Veröffentlichungsnummer: **0 065 997**

**A1**

(12) ## EUROPÄISCHE PATENTANMELDUNG

(21) Anmeldenummer: **81104134.2**

(22) Anmeldetag: **29.05.81**

(51) Int. Cl.³: **A 61 N 1/16**

(43) Veröffentlichungstag der Anmeldung:
**08.12.82 Patentblatt 82/49**

(84) Benannte Vertragsstaaten:
**AT BE CH DE FR GB IT LI LU NL SE**

(71) Anmelder: **Przedsiebiorstwo Wdrazania i
Upowszechniania Postepu Technicznego i
Organizacyjnego "POSTEOR" we Wroclawiu
ul. Krasinskiego 1
50-954 Wroclaw(PL)**

(72) Erfinder: **Szul, Roman
ul. Bezpieczna 89/10
Wroclaw(PL)**

(72) Erfinder: **Szul, Marek
ul. Kosciuszki 40/3
Wroclaw(PL)**

(72) Erfinder: **Krukowski, Marek
ul. Wejherowska 3/6
Wroclaw(PL)**

(74) Vertreter: **Finck, Dieter et al,
Patentanwälte v. Füner, Ebbinghaus, Finck
Mariahilfplatz 2 & 3
D-8000 München 90(DE)**

(54) **Verfahren und Anordnung zum Schutz von lebenden Organismen vor Wasserstrahlung.**

(57) Die Erfindung löst die Frage des Schutzes von lebenden Organismen vor Wasserstrahlung. Im Bereich 1 wird die Anwesenheit von Metallatome enthaltenden Körpern festgestellt, die für die Neutralisierung der schädlichen Wasserstrahlung ausgenutzt werden. Vorzugsweise sind dies Elektroanlagen 2, Wasserversorgungsanlagen oder die Gebäudebewehrung. Die Anlage 2 wird galvanisch oder feldmäßig mit einem Generator 3 über eine Spule 4 gekoppelt und in Zeitabständen von einer Stunde ca. 10 s lang mit Impulsen erregt. Die Anordnung besteht aus einem elektrischen Impulsgenerator 3, wobei die Zeitdauer der Impulse proportional zum Kehrwert der freien Magnetmoment-Präzessionsfrequenz der submolekularen Elemente des Metalles ist, aus dem die Anlage 2 aufgebaut ist, die mit der Spule 4 gekoppelt wird.

Beschreibung:

Gegenstand der Erfindung ist ein Verfahren und eine Anordnung zum Schutz von lebenden Organismen vor Wasserstrahlung, bestimmt für den allgemeinen Einsatz, um den für Menschen und Tiere ungünstigen Einfluß von Wasserstrahlung auszuscheiden, die von unter Räumen liegenden Wasseradern abgestrahlt wird, in welchen Menschen oder Tiere leben.

Allgemein bekannt ist der ungünstige Einfluß von Wasseraderstrahlungen, besonders aber die schädliche Einwirkung von Wasserstrahlung in Bereichen, in denen sich Wasseradern schneiden.

Aus der DE-PS 2 212 990 ist eine Anordnung zum Schutz vor Wasseraderstrahlung bekannt, die aus einer Reihenschaltung von zwei kernlosen Spulen besteht, die wiederum mit zwei Spulen mit Kernen verbunden sind, deren andere Enden mit zwei Kondensatoren verbunden sind. Die beiden in Reihe geschalteten Kondensatoren sind parallel zu einem Widerstand geschaltet. Aus durchgeführten Untersuchungen geht hervor, daß es die bekannte Lösung nicht gestattet, einfach und vor allem wirkungsvoll die Wasserstrahlung von unter Räumen liegenden Wasseradern auszuscheiden, in denen Menschen und/oder Tiere leben. Das erfindungsgemäße Schutzverfahren von lebenden Organismen vor Wasserstrahlung beruht darauf, daß ein Metallatome enthaltender und im zu schützenden Bereich angeordneter Körper, vorzugsweise eine in diesem Bereich befindliche metallische Elektroanlage, Wasserversorgungsanlage oder die konstruktive Bewehrung eines Gebäudes galvanisch oder feldmäßig mit einem elektrischen Gebergerät gekuppelt wird. Dann wird die genannte Anlage aus dem Gebergerät dauernd oder periodisch mit elektrischen Impulsen versorgt, deren Zeitdauer zum Kehrwert der freien Präzessionsfrequenz des Magnetmomentes des Atomkernes desjenigen Metalles proportional ist, aus dem die Anlage aufgebaut ist, oder auch mit Elektroimpulsen, deren Zeitdauer zum Kehrwert der freien Präzessionsfrequenz des Magnetmomentes der submolekularen Elemente des erwähnten Metalles proportional ist, aus dem die erwähnte Anlage aufgebaut ist.

Die erfindungsgemäße Anordnung zum Schutz lebender Organismen vor Wasserstrahlung besteht aus einem elektrischen Impulsgenerator, der galvanisch oder feldmäßig mit einer metallischen Elektro-Wasserversorgungsanlage bzw. mit der Bewehrung des im zu schützenden Bereich angeordneten Gebäudes angeordnet ist. Die erfindungsgemäße Lösung schließt in einfacher Art und Weise die ungünstige Strahlung von Wasseradern sogar im Falle von sehr großen Wohn-, Wirtschafts- oder öffentlichen Gebäuden aus.

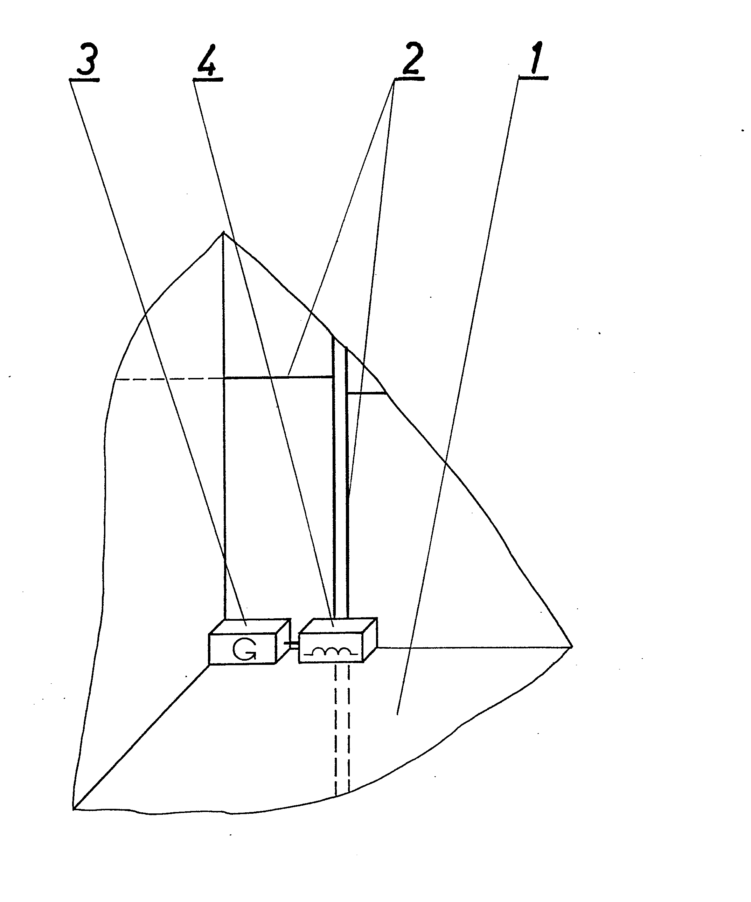

Die Erfindung wird anhand des in der Zeichnung dargestellten Ausführungsbeispiels näher erläutert. Die Zeichnung zeigt ein Blockschaltbild der Schutzanordnung.

In dem vor Wasserstrahlung zu schützenden Bereich 1, unter dem sich z.B. Wasseradern schneiden, wird die Anwesenheit von Metallatome enthaltenden Körpern festgelegt, die für die Neutralisierung der schädlichen Wasserstrahlung genutzt werden. Vorzüglich eignen sich dazu Elektroanlagen 2, Wasserversorgungsanlagen oder eine im zu schützenden Bereich 1 angeordnete konstruktive Bewehrung des Gebäudes. Die Elektroanlage 2 wird galvanisch oder feldmäßig mit einem elektrischen Impulsgenerator 3 gekuppelt. Im Falle einer Elektroanlage 2 wird vorzugsweise eine magnetische Kopplung mit dem Impulsgenerator 3 mittels einer Spule 4 gewählt. Darauf wird die Anlage 2 aus dem Impulsgenerator 3 über die Spule 4 in Zeitabständen von einer Stunde ca. 10 s lang periodisch mit Impulsen versorgt, deren Zeitdauer proportional zum Kehrwert der freien Magentmoment-Präzessionsfrequenz des Atomkernes desjenigen Metalles ist, aus dem die Anlage 2 aufgebaut ist, oder aber auch mit Impulsen, deren Zeitdauer proportional zum Kehrwert der freien Magnetmoment-Präzessionsfrequenz der submolekularen Elemente desjenigen Metalles ist, aus dem die Anlage 2 aufgebaut ist. Die Anlage 2 kann aus dem Generator 3 auch dauernd versorgt werden, obwohl aus durchgeführten Versuchen hervorgeht, daß die periodische Versorgung der Anlage 2 günstiger ist. Die Impulsdauer $T_s$ wird durch die Beziehung:

$$T_s = k \cdot \frac{1}{\nu}$$

beschrieben, wobei:

$\nu$ die freie Präzessionsfrequenz des Atomkernes des Metalles ist, das den Grundbestandteil der Legierung bildet, aus dem die Anlage 2 aufgebaut ist, wobei

$$\nu = \frac{\mu_m \cdot H_o}{h}$$

mit $\mu_m$ = Magnetmoment des Metallatomes
$H_o$ = Erdmagnetfeldstärke
$h$ = Planck'sche Konstante.

Der Proportionalitätsfaktor $k$ ist eine von der Form der elektrischen Impulse des Generators 3 abhängige Größe; er beträgt für Rechteckimpulse $k = 1$. Falls die Anlage 2 aus Aluminium aufgebaut ist, beträgt die Impulsdauer für Rechteckimpulse:

- 4 -

$$T_s = 1 \cdot \frac{h}{\mu_{Al} \cdot H_o} = 0,3855 \text{ ms}$$

Das Tastverhältnis der Impulse ist keine kritische Größe; sie beträgt vorzugsweise unter 50 %. Die erfindungsgemäße Anordnung besteht aus einem elektrischen Impulsgenerator 3, der magnetisch über die Spule 4 mit einer Elektroanlage 2 gekoppelt ist, die im vor Wasserstrahlung geschützten Bereich 1 angeordnet ist, unter dem Wasseradern verlaufen. Als Anlage 2 kann auch die Wasserversorgungsanlage oder die im Schutzbereich 1 angeordnete Bewehrung des Gebäudes benutzt werden.

Die Kopplung des Generators 3 mit der Anlage 2 kann feldmäßig mittels Magnetfeld, elektrisch oder elektromagnetisch ausgeführt werden. Der Generator 3 bildet die Quelle von elektrischen Impulsen, deren Zeitdauer proportional zum Kehrwert der freien Magnetmoment-Präzessionsfrequenz des Atomkernes desjenigen Metalles ist, aus dem die Anlage 2 aufgebaut ist oder auch die Quelle von elektrischen Impulsen, deren Zeitdauer proportional zum Kehrwert der freien Magnetmoment-Präzessionsfrequenz der submolekularen Elemente des erwähnten Metalles ist, aus dem die Anlage 2 aufgebaut ist. Der Proportionalitätsfaktor k ist abhängig von der Form der Impulse des Generators 3 und beträgt für Rechteckimpulse k = 1. Bei einer feldmäßigen Kopplung des Generators 3 mit der Anlage 2 wird das Feld der Spule 4 ausgenutzt.

PATENTANWÄLTE

SCHIFF v. FÜNER STREHL SCHÜBEL-HOPF EBBINGHAUS FINCK

MARIAHILFPLATZ 2 & 3, MÜNCHEN 90
POSTADRESSE: POSTFACH 95 01 60, D-8000 MÜNCHEN 95

ALSO PROFESSIONAL REPRESENTATIVES
BEFORE THE EUROPEAN PATENT OFFICE

KARL LUDWIG SCHIFF (1964-1978)
DIPL. CHEM DR. ALEXANDER v. FÜNER
DIPL. ING. PETER STREHL
DIPL. CHEM. DR. URSULA SCHÜBEL-HOPF
DIPL. ING. DIETER EBBINGHAUS
DR. ING. DIETER FINCK

TELEFON (089) 48 20 54
TELEX 5-23565 AURO D
TELEGRAMME AUROMARCPAT MÜNCHEN

**0065997**

PRZEDSIEBIORSTWO WDRAZANIA

I UPOWSZECHNIANIA POSTEPU

TECHNICZNEGO I ORGANIZACYJNEGO

"POSTEOR" WE WROCŁAWIU

29. Mai 1981

EPA-21986

Verfahren und Anordnung zum Schutz von
lebenden Organismen vor Wasserstrahlung

Patentansprüche:

1. Verfahren zum Schutz von lebenden Organismen vor Wasserstrahlung, dadurch g e k e n n z e i c h n e t , daß ein Metallatome enthaltender und im zu schützenden Bereich (1) angeordneter Körper, vorzugsweise eine in diesem Bereich bestehende metallische Elektroanlage (2), Wasserversorgungsanlage oder die konstruktive Gebäudebewehrung galvanisch oder feldgemäß mittels Magnetfeld, elektrischem oder elektromagnetischem Feld mit einem elektrischen Impulsgenerator (3) gekoppelt wird und daß darauf die Anlage (2) dauernd oder periodisch aus dem Generator (3) mit Elektroimpulsen versorgt wird.

2. Anordnung zum Schutz von lebenden Organismen vor Wasserstrahlung, g e k e n n z e i c h n e t durch einen elektrischen Impulsgenerator (3), der galvanisch oder feldmäßig mit der im zu schützenden Bereich (1) angeordneten Elektroanlage (2), Wasserversorgungsanlage oder Gebäudebewehrung gekoppelt ist, wobei der Generator (3) elektrische Impulse erzeugt, deren Zeitdauer proportional zum Kehrwert der freien Magnetmoment-Präzessionsfrequenz des Atomkernes desjenigen Metalles ist, aus dem die Anlage (2) aufgebaut ist, oder elektrische Impulse, deren Zeitdauer proportional zum Kehrwert der freien Magnetmoment-Präzessionsfrequenz der submolekularen Atomelemente desjenigen Metalles ist, aus dem die Anlage (2) aufgebaut ist.

## EUROPÄISCHER RECHERCHENBERICHT

Europäisches
Patentamt

| EINSCHLÄGIGE DOKUMENTE | | | |
|---|---|---|---|
| Kategorie | Kennzeichnung des Dokuments mit Angabe, soweit erforderlich, der maßgeblichen Teile | betrifft Anspruch | |
| A | FR - A - 1 517 678 (MONJAUX)<br>* Seite 1, rechte Spalte, Abschnitt 3; Seite 2, linke Spalte, Abschnitt 3 un rechte Spalte, Abschnitt 5 * | 1,2 | |
| A | DE - A - 1 514 373 (RANSCHT)<br>* Seite 2, Zeilen 4-21 * | 1,2 | |
| A | FR - A - 972 232 (SCHNEIDER)<br>* Seite 1, linke Spalte * | 1,2 | |
| A | FR - A - 2 178 242 (EXQUISIT)<br>* Seite 1, Zeile 33 - Seite 2, Zeile 19 * | 1,2 | |
| A | GB - A - 415 694 (DANNERT)<br>* Seite 1, Zeilen 33-82 * | 1,2 | |

**KLASSIFIKATION DER ANMELDUNG (Int. Cl.³)**

A 61 N 1/16

**RECHERCHIERTE SACHGEBIETE (Int. Cl.³)**

A 61 N 1/16
5/00

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 29-01-1982 | SIMON |

EPA form 1503.1 06.78